# EUROPEAN PATENT APPLICATION

(11) **EP 4 810 254 A1**
(43) Date of publication of application: **23.09.2026**
(21) Application number: 26163181.6
(22) Date of filing: 09.03.2026
(51) Int. Cl.: B41F 33/00, B65B 59/04

(54) **COMPONENT IDENTIFICATION SYSTEM OF A PACKAGING MACHINE**

(30) Priority: 17.03.2025 IT 202500005361
(71) Applicant: GDM S.p.A., 40133 Bologna (IT)
(72) Inventor: ZAVALLONI, Alessandro, 40133 Bologna (IT); CARUANA, Paolo, 40133 Bologna (IT)
(74) Representative: Emmi, Mario

(57) **Abstract**

Machine (1) and related method for producing and/or packaging products made from cellulose or absorbent products; said machine (1) comprising at least one component (2) comprising identification means (3, 3') connected or joined to said at least one component (2) and at least one identification device (4) configured to detect at least one or more of said identification means (3).

## Description

### Technical field of the invention

The present invention relates to a machine for producing and/or packaging products made from cellulose or absorbent products, such absorbent products preferably comprising nappies, adult diapers, sanitary pads, drawsheets and plasters.

Purely by way of non-limiting example, such a machine for producing and/or packaging products made from cellulose or absorbent products finds advantageous application for absorbent products preferably comprising nappies, diapers, sanitary pads, drawsheets and plasters.

Moreover, purely by way of non-limiting example, such a machine for producing and/or packaging products made from cellulose or absorbent products finds advantageous application for cellulose-based products treated with additives and moulded to make typically disposable products with reduced absorbency to withstand moisture.

The present invention also relates to a method for producing and/or packaging products made from cellulose or absorbent products, such absorbent products preferably comprising nappies, adult diapers, sanitary pads, drawsheets and plasters.

Purely by way of non-limiting example, said method for producing and/or packaging products made from cellulose or absorbent products finds advantageous application for absorbent products preferably comprising nappies, diapers, sanitary pads, drawsheets and plasters.

Purely by way of non-limiting example, said method for producing and/or products made from cellulose or absorbent products finds advantageous application for cellulose-based products treated with additives and moulded to make typically disposable products with reduced absorbency to withstand moisture.

### State of the art:

As is well known, in recent years, technological development for producing and/or packaging products on an industrial scale by means of automatic machines, particularly in the field of tobacco products, has focused attention on the recognition of the originality or compatibility of machine parts (i.e. parts manufactured or supplied by a manufacturer) that can be replaced due to wear, malfunctioning or because they are specific to format change or size change for producing and/or packaging specific products.

In particular, identification or recognition systems are known whereby each replaceable machine element comprises a non-removable passive element that stores at least one identification code. The system further comprises a number of detectors each adapted to interrogate a respective passive element and detect the respective identification codes; finally, the system comprises a processing unit adapted to compare each detected identification code with identification codes (previously set and stored therein) of original components.

However, said recognition systems have several drawbacks.

In particular, a first drawback is the fact that said systems typically have a large number of detectors (one for each passive element), leading to a problem of a large footprint that is difficult to reconcile with the internal spaces of typical machines for producing and/or packaging products made from cellulose or absorbent products.

In addition, a second drawback is that said systems present reliability problems, even and especially when the size of the entire machine becomes significant (in particular, the length) as is typically the case with machines for producing and/or packaging products made from cellulose or absorbent products; these reliability problems are often attributable to the large number of detectors involved.

Finally, a third drawback is the fact that said systems have an intrinsic implementation difficulty, both on new and existing machines, largely due to the large number of detectors present.

### Summary of the invention:

The object of the present invention is to provide a machine for producing and/or packaging products made from cellulose or absorbent products that overcomes the drawbacks of the above-mentioned prior art.

In particular, it is the purpose of the present invention to provide a machine for producing and/or packaging products made from cellulose or absorbent products that ensures that each component to be identified can be reliably detected, in a simple manner (on both new and existing machines) and avoiding machine footprint issues.

Furthermore, it is the purpose of the present invention to provide a method for producing and/or packaging products made from cellulose or absorbent products that overcomes the drawbacks of the above-mentioned prior art.

In particular, it is the purpose of the present invention to provide a method for producing and/or packaging products made from cellulose or absorbent products that ensures that each component to be identified can be reliably detected, in a simple manner (on both new and existing machines) and avoiding machine footprint issues.

According to the present invention, a machine 1 for producing and/or packaging products made from cellulose or absorbent products, as defined in claim 1, is provided.

Purely by way of non-limiting example, said machine finds advantageous application for absorbent products preferably comprising nappies, diapers, sanitary pads, drawsheets and plasters.

Moreover, purely by way of non-limiting example, such a machine for producing and/or packaging products made from cellulose or absorbent products finds advantageous application for cellulose-based products treated with additives and moulded to make typically disposable products with reduced absorbency to withstand moisture.

Preferably, said machine comprises at least one component 2.

The term said at least one component 2 refers to any component, mechanical or electronic, that can be part of a production and/or packaging line for products made from cellulose or absorbent products, such as a component of a sealing unit, a cutting unit, a folding unit, a conveying unit, a stacking unit, a bagging unit, a pressing unit, a forming unit.

Preferably, each component 2 to be identified comprises identification means (3, 3') connected or joined to said at least one component 2.

The term identification means (3, 3') refers to any element or group of elements configured to give each component 2 a univocal identification.

Said identification means (3, 3') can be connected with physical contact to a respective component 2 in any manner, or can be connected without physical contact to a respective physical component in any manner (e.g. wireless).

Preferably, said machine 1 comprises at least one identification device 4 configured to detect at least one or more of said identification means (3, 3').

More specifically, preferably, an identification device 4 is thus configured to detect even more than one of said identification means (3, 3') also distributed over several components (2).

This solution solves all the aforementioned technical drawbacks.

In particular, the presence of said at least one identification device 4 configured to detect at least one or more of said identification means (3, 3') makes it possible to significantly reduce the number of detectors provided to identify each component 2; this ensures that each component to be identified is detected reliably, easily (both on new and existing machines) and avoiding machine footprint issues.

In particular, precisely in the case in which said identification means (3, 3') are present on more than one component (2), this solution achieves the intended purposes, since a single identification device (4) can also identify several identification means (3, 3') present on several components (2), in fact, identifying several components (2).

This makes it possible to considerably reduce the number of overall recognition elements, thus simplifying the structure considerably, especially in the case of very long and complex machines.

According to the present invention, a method for producing and/or packaging products made from cellulose or absorbent products, as defined in claim 11, is also provided.

Purely by way of non-limiting example, said method finds advantageous application for absorbent products preferably comprising nappies, diapers, sanitary pads, drawsheets and plasters.

Moreover, purely by way of non-limiting example, said method for producing and/or packaging products made from cellulose or absorbent products finds advantageous application for cellulose-based products treated with additives and moulded to make typically disposable products with reduced absorbency to withstand moisture.

Preferably, said method comprises a step of providing at least one operating component 2 of a machine 1.

The term said at least one operating component 2 refers to any component, mechanical or electronic, that can be part of a production and/or packaging line for products made from cellulose or absorbent products, such as a component of a sealing unit, a cutting unit, a folding unit, a conveying unit, a stacking unit, a bagging unit, a pressing unit, a forming unit.

Preferably, each component 2 to be identified comprises identification means (3, 3') connected or joined to said at least one component 2.

The term identification means (3, 3') refers to any element or group of elements configured to give each component 2 a univocal identification.

Said identification means (3, 3') can be connected with physical contact to a respective component 2 in any manner, or can be connected without physical contact to a respective physical component in any manner (e.g. wireless).

Preferably, said method comprises a step of providing at least one identification device 4 configured to detect at least one of said identification means (3, 3').

This solution solves all the aforementioned technical drawbacks.

In particular, said step of providing at least one identification device 4 configured to detect at least one of said identification means (3, 3') makes it possible to significantly reduce the number of detectors provided to identify each component 2; this ensures that each component to be identified is detected reliably, easily (both on new and existing machines) and avoiding machine footprint issues.

The machine 1 configured according to the present invention, and the method thereof, meet the need to automatically identify components (preferably mechanical or electronic) mounted on at least one component of said machine for the production and/or industrial packaging of products made from cellulose or absorbent products. Said at least one component may be a component suitable for producing and/or packaging any products made from cellulose or absorbent products, or said at least one component may be a specific component that must be replaced in the event of a format or size change for producing and/or packaging related products made from cellulose or absorbent products.

The invention also finds advantageous application in environments where detection is difficult in dusty, unlit or confined spaces.

In other words, said machine (and the related method), which operates in alternating motion (i.e., the operating phase includes stops) or continuous motion (i.e., the operating phase does not include any stops), is configured to recognize which components have been assembled and to verify that these components are actually supplied or produced and approved by the manufacturer and/or compatible with the format or size of the product made from cellulose or absorbent product to be made and/or packaged.

### Brief description of the drawings:

For a better understanding of the present invention, a preferred embodiment is now described, by way of non-limiting example only, with reference to the attached drawing, in which:

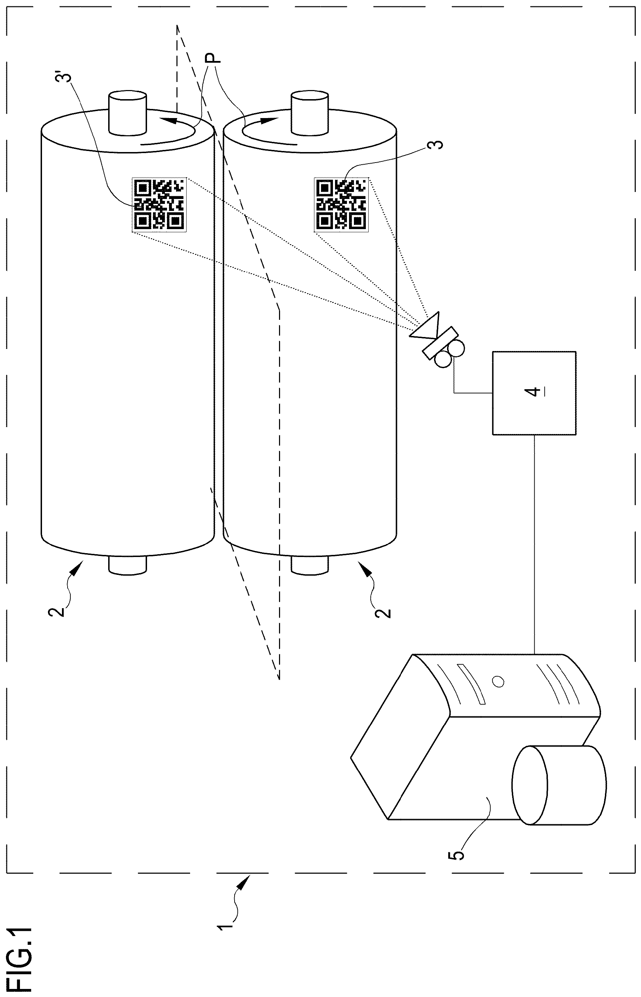

Figure 1 shows a schematic view of a machine or portion thereof for producing and/or packaging products made from cellulose or absorbent products according to the invention; in particular, Figure 1 shows the non-limiting example case of two distinct components 2 of the machine 1 (e.g. two counter-rotating rollers, but they could obviously be in any number of components and not necessarily in the form of rollers) to which, for each one of them, a specific identification means (3, 3') is applied and with a single identification device 4 capable of reading both identification means (3, 3').

### Detailed description of the invention:

Referring to Figure 1, a machine 1 or a portion 1 thereof is schematically shown for illustrative and non-limiting purposes only. Said machines for producing and/or packaging products made from cellulose or absorbent products, said absorbent products preferably comprising nappies, adult diapers, sanitary pads, drawsheets and plasters, are in themselves well known and are therefore not described here in detail in the reference drawing. Therefore, the drawing schematically represents the new part integrated/to be integrated into any of these machines.

This machine 1 finds advantageous application for absorbent products preferably comprising nappies, diapers, sanitary pads, drawsheets and plasters.

Moreover, purely by way of non-limiting example, such a machine 1 for producing and/or packaging products made from cellulose or absorbent products finds advantageous application for cellulose-based products treated with additives and moulded to make typically disposable products with reduced absorbency to withstand moisture.

Preferably, said machine 1 comprises at least one component 2 (two separate components 2 in the form of two counter-rotating rollers are shown by way of example in Figure 1).

The term said at least one component 2 refers to any component, mechanical or electronic, that can be part of a production and/or packaging line for products made from cellulose or absorbent products, such as a component of a sealing unit, a cutting unit, a folding unit, a conveying unit, a stacking unit, a bagging unit, a pressing unit, a forming unit.

Said at least one component 2 can be specific to the type of products being processed by the production and/or packaging machine, i.e. said at least one component 2 is of a format compatible with the type of products to be made and/or packaged. In other words, the component 2 can be replaced by another component 2 whenever the format or size of the products being processed and/or packaged changes.

Preferably, each component 2 to be identified comprises identification means (3, 3') connected or joined to said at least one component 2. In the example case of Figure 1, in fact, one component 2 comprises identification means 3 and the other component 2 comprises identification means (3').

The term identification means (3, 3') refers to any element or group of elements configured to give each component 2 a univocal identification.

Said identification means (3, 3') can be connected with physical contact to a respective component 2 in any manner, or can be connected without physical contact to a respective physical component in any manner (e.g. wireless).

Preferably, said machine comprises at least one identification device 4 configured to detect at least one or more of said identification means (3, 3').

Depending on the distance at which the components 2 are placed from each other, it is thus possible to use a single identification device to simultaneously detect several identification means (3, 3'). The example in Figure 1 shows the case of identification, in fact, of two identification means (3, 3') each placed on a specific component (2).

This greatly reduces the complexity of the machine integrating this solution.

These characteristics derive in fact from the experimental tests carried out by the Applicant, in which the Applicant tried out different alternative solutions, in order to identify the solution that best guaranteed detecting each component to be identified reliably, easily (both on new and existing machines) and avoiding machine footprint issues.

In particular, the presence of said at least one identification device 4 configured to detect at least one or more of said identification means (3, 3') makes it possible to significantly reduce the number of detectors provided to identify each component 2; this ensures that each component to be identified is detected reliably, easily (both on new and existing machines) and avoiding machine footprint issues.

In detail, said at least one identification device 4 is configured to detect one or more of said identification means (3, 3'), which allows to significantly limit the number of said at least one identification device 4, thus being able to identify more than one component 2 of said machine 1.

According to a further aspect of the invention, said identification means (3, 3') are univocal for each component 2. In other words, each component 2 to be identified has a respective and univocal identification means (3, 3').

Preferably, said identification means (3, 3') are configured in such a way that said at least one identification device 4 generates a univocal detection signal for each component 2 adapted to recognize each component 2.

In other words, said identification means (3, 3') are detectable by said at least one identification device 4 and are configured in such a way that each detection signal of each component 2 to be identified is univocal for each component 2. In this manner, said at least one identification device 4 is capable of recognizing/identifying at least one component 2 of the machine 1 presenting said identification means (3, 3').

This feature was identified by the Applicant as the solution that best guaranteed detecting each component to be identified reliably, easily (both on new and existing machines) and avoiding machine footprint issues.

According to a further aspect of the invention, said machine 1 comprises at least one control and command unit 5.

Preferably, said control and command unit 5 is configured to store beforehand each univocal detection signal for each component 2 and to associate each univocal detection signal, subsequently detected, with each component 2. In other words, said control and command unit 5 is any conventional electronic device/system capable of storing a respective univocal detection signal for each component 2 to be identified, by means of an instruction step typically carried out by the manufacturer or supplier of said at least one component 2; further, in an operating step subsequent to said instruction step, said control and command unit 5 is capable of associating each univocal detection signal with each component.

This feature was identified by the Applicant as the solution that best guaranteed detecting each component to be identified reliably, easily (both on new and existing machines) and avoiding machine footprint issues.

According to a further aspect of the invention, said at least one control and command unit 5 is configured to act on at least one component of the machine for stopping or preventing its functioning, if said control and command unit 5 does not detect at least one detection signal stored beforehand or detects at least one detection signal not stored beforehand. In other words, if said control and command unit 5 does not detect at least one detection signal stored beforehand (for example due to the lack of at least one component 2 compatible for the specific production and/or packaging or due to the lack of at least one component 2 provided by a manufacturer or supplier) or detects at least one detection signal not stored beforehand (for example due to the presence of at least one component 2 not compatible for the specific production and/or packaging or due to the presence of at least one component 2 not provided by a manufacturer or supplier), said control and command unit 5 is able to act on the at least one component (which may be the same comprising said identification means 3 or may be a different component) of the machine 1 in order to stop it (in case the machine 1 was operating during the assembly or replacement of said at least one component 2) or not to start its operation (in case the machine 1 was in a stopped state during the assembly or replacement of said at least one component 2).

Alternatively or additionally, said at least one control and command unit 5 is configured to send an alarm signal to a device locally or remotely, if said control and command unit 5 does not detect at least one detection signal stored beforehand or detects at least one detection signal not stored beforehand. In other words, if said control and command unit 5 does not detect at least one detection signal stored beforehand (e.g. due to the lack of said at least one component 2 compatible for the specific production and/or packaging or due to the lack of said at least one component 2 provided by a manufacturer or supplier) or detects at least one detection signal not stored beforehand (e.g. due to the presence of said at least one component 2 not compatible for the specific production and/or packaging or due to the presence of said at least one component 2 not provided by a manufacturer or supplier), said control and command unit 5 is able to send an alarm signal to a device (of any electronic type, also in the cloud) locally or remotely to said manufacturer or supplier of said at least one component 2 (and/or to the manufacture in which said machine 1 is present), so as to advise that on the machine 1 the at least one component 2 not compatible for the specific production and/or packaging or the at least one component 2 not supplied by a manufacturer or supplier has been assembled.

Those features, additionally or alternatively, of said at least one control and command unit 5 are designed by the Applicant to ensure reliable identification of each component 2 which can also be controlled remotely, in particular by the manufacturer or supplier of said at least one component 2 of the machine 1.

According to a further aspect of the invention, said at least one component 2 is movable along a predetermined path P. In other words, said at least one component 2 can be a motionless component during its operation or it can be movable during its operation, describing its mobility along a predetermined path P.

According to a further aspect of the invention, said at least one identification device 4 is arranged facing said at least one component 2 and/or said path P. In other words, said at least one identification device 4 may be positioned at or near said at least one component 2 comprising said identification means (3, 3') and/or may be positioned at or near said path P of said at least one component 2 comprising said identification means (3, 3').

This feature was identified by the Applicant as the solution that best guaranteed detecting each component to be identified reliably, easily (both on new and existing machines) and avoiding machine footprint issues.

According to a further aspect of the invention, said identification means (3, 3') are applied in a removable way to each component 2 or are integrated into each component 2. In other words, said identification means (3, 3') are either applicable and removable to each component 2 to be identified or are an integral part of each component to be identified.

This feature was identified by the Applicant as the solution that best guaranteed detecting each component to be identified reliably, easily (both on new and existing machines) and avoiding machine footprint issues.

According to a further aspect of the invention, said at least one identification device 4 comprises at least one sensor selected from the group consisting of: inductive sensor, radio frequency sensor (e.g. RFID), optical sensor, mechanical sensor, thermal sensor, proximity sensor, movement sensor.

According to a further aspect of the invention, said identification means (3, 3') comprise at least one element selected from the group consisting of: barcode, passive electronic devices (e.g. RFID), alphanumeric sequences.

According to a further aspect of the invention, said method for producing and/or packaging products made from cellulose or absorbent products comprises a step of providing at least one operating component 2 of a machine 1 for producing and/or packaging products made from cellulose or absorbent products.

The term said at least one operating component 2 refers to any component, mechanical or electronic, that can be part of a production and/or packaging line for products made from cellulose or absorbent products, such as a component of a sealing unit, a cutting unit, a folding unit, a conveying unit, a stacking unit, a bagging unit, a pressing unit, a forming unit.

Preferably, each component 2 to be identified comprises identification means (3, 3') connected or joined to said at least one component 2.

The term identification means (3, 3') refers to any element or group of elements configured to give each component 2 a univocal identification.

Said identification means (3, 3') can be connected with physical contact to a respective component 2 in any manner, or can be connected without physical contact to a respective physical component in any manner (e.g. wireless).

Preferably, said method comprises a step of providing at least one identification device 4 configured to detect at least one of said identification means (3, 3').

These characteristics derive from the experimental tests carried out by the Applicant, in which the Applicant tried out different alternative solutions, in order to identify the solution that best guaranteed detecting each component to be identified reliably, easily (both on new and existing machines) and avoiding machine footprint issues.

In particular, the step of providing said at least one identification device 4 configured to detect at least one of said identification means (3, 3') makes it possible to significantly reduce the number of detectors provided to identify each component 2; this ensures that each component to be identified is detected reliably, easily (both on new and existing machines) and avoiding machine footprint issues.

In detail, said at least one identification device 4 is configured to detect one or more of said identification means (3, 3'), which allows to significantly limit the number of said at least one identification device 4, thus being able to identify more than one component 2.

According to a further aspect of the invention, said identification means (3, 3') are univocal for each component 2. In other words, each component 2 to be identified has a respective and univocal identification means (3, 3').

Preferably, said identification means (3, 3') are configured in such a way that said at least one identification device 4 generates a univocal detection signal for each component 2 adapted to recognize each component 2.

In other words, said identification means (3, 3') are detectable by said at least one identification device 4 and are configured in such a way that each detection signal of each component 2 to be identified is univocal for each component 2. In this manner, said at least one identification device 4 is capable of recognizing/identifying at least one component 2 of the machine 1 presenting said identification means (3, 3').

This feature was identified by the Applicant as the solution that best guaranteed detecting each component to be identified reliably, easily (both on new and existing machines) and avoiding machine footprint issues.

According to a further aspect of the invention, said method comprises a step of providing at least one control and command unit 5.

Preferably, said control and command unit 5 is configured to store beforehand each univocal detection signal for each component 2 and to associate each univocal detection signal, subsequently detected, with each component 2. In other words, said control and command unit 5 is any conventional electronic device/system capable of storing a respective univocal detection signal for each component 2 to be identified, by means of an instruction step typically carried out by the manufacturer or supplier of said at least one component 2; further, in an operating step subsequent to said instruction step, said control and command unit 5 is capable of associating each univocal detection signal with each component.

This feature was identified by the Applicant as the solution that best guaranteed detecting each component to be identified reliably, easily (both on new and existing machines) and avoiding machine footprint issues.

According to a further aspect of the invention, said at least one control and command unit 5 is configured to act on at least one component of the machine 1 (for the production and/or packaging of cellulose-based products or absorbent products) for stopping or preventing its functioning, if said control and command unit 5 does not detect at least one detection signal stored beforehand or detects at least one detection signal not stored beforehand. In other words, if said control and command unit 5 does not detect at least one detection signal stored beforehand (for example due to the lack of at least one component 2 compatible for the specific production and/or packaging or due to the lack of at least one component 2 provided by a manufacturer or supplier) or detects at least one detection signal not stored beforehand (for example due to the presence of at least one component 2 not compatible for the specific production and/or packaging or due to the presence of at least one component 2 not provided by a manufacturer or supplier), said control and command unit 5 is able to act on the at least one component (which may be the same comprising said identification means 3 or may be a different component) of the machine 1 in order to stop it (in case the machine 1 was operating during the assembly or replacement of said at least one component 2) or not to start its operation (in case the machine 1 was in a stopped state during the assembly or replacement of said at least one component 2).

Alternatively or additionally, said at least one control and command unit 5 is configured to send an alarm signal to a device locally or remotely, if said control and command unit 5 does not detect at least one detection signal stored beforehand or detects at least one detection signal not stored beforehand. In other words, if said control and command unit 5 does not detect at least one detection signal stored beforehand (e.g. due to the lack of said at least one component 2 compatible for the specific production and/or packaging or due to the lack of said at least one component 2 provided by a manufacturer or supplier) or detects at least one detection signal not stored beforehand (e.g. due to the presence of said at least one component 2 not compatible for the specific production and/or packaging or due to the presence of said at least one component 2 not provided by a manufacturer or supplier), said control and command unit 5 is able to send an alarm signal to a device (of any electronic type, also in the cloud) locally or remotely to said manufacturer or supplier of said at least one component 2 (and/or to the manufacture in which said machine 1 is present), so as to advise that on the machine 1 the at least one component 2 not compatible for the specific production and/or packaging or the at least one component 2 not supplied by a manufacturer or supplier has been assembled.

Those features, additionally or alternatively, of said at least one control and command unit 5 are designed by the Applicant to ensure reliable identification of each component 2 which can also be controlled remotely, in particular by the manufacturer or supplier of said at least one component 2 of the machine 1.

According to a further aspect of the invention, said at least one component 2 is movable along a predetermined path P. In other words, said at least one component 2 can be a motionless component during its operation or it can be movable during its operation, describing its mobility along a predetermined path P.

According to a further aspect of the invention, said at least one identification device 4 is arranged facing said at least one component 2 and/or said path P. In other words, said at least one identification device 4 may be positioned at or near said at least one component 2 comprising said identification means (3, 3') and/or may be positioned at or near said path P of said at least one component 2 comprising said identification means (3, 3').

This feature was identified by the Applicant as the solution that best guaranteed detecting each component to be identified reliably, easily (both on new and existing machines) and avoiding machine footprint issues.

According to a further aspect of the invention, said identification means (3, 3') are applied in a removable way to each component 2 or are integrated into each component 2. In other words, said identification means (3, 3') are either applicable and removable to each component 2 to be identified or are an integral part of each component to be identified.

This feature was identified by the Applicant as the solution that best guaranteed detecting each component to be identified reliably, easily (both on new and existing machines) and avoiding machine footprint issues.

The machine 1 for producing and/or packaging products made from cellulose or absorbent products, and the related method, as described above, make it possible to achieve the purposes stated above, such as ensuring that each component to be identified can be reliably detected, in a simple manner (on both new and existing machines) and avoiding machine footprint issues.

Finally, it is clear that the machine 1 for producing and/or packaging products made from cellulose or absorbent products, and the method thereof, described and illustrated herein, may be modified and varied without departing from the protective scope of the present invention as defined in the appended claims.

## Claims

1. Machine (1) for producing and/or packaging products made from cellulose or absorbent products, said absorbent products preferably comprising nappies, adult diapers, sanitary pads, drawsheets and plasters, said machine comprising at least one component (2) comprising identification means (3, 3') connected or joined to said at least one component (2) and at least one identification device (4) configured to detect at least one or more of said identification means (3, 3').

2. Machine according to the previous claim, wherein said identification means (3, 3') are univocal for each component (2) and configured in such a way that said identification device (4) generates a univocal detection signal for each component (2) adapted to recognize each component (2).

3. Machine according to one or more of the previous claims, wherein said machine comprises at least one control and command unit (5) configured to store beforehand each univocal detection signal for each component (2) and to associate each univocal detection signal, subsequently detected, to each component (2).

4. Machine according to claim 3, wherein said at least one control and command unit (5) is configured to act on at least one component of the machine (1) for stopping or preventing its functioning, if said control and command unit (5) does not detect at least one detection signal stored beforehand or detects at least one detection signal not stored beforehand.

5. A machine according to one or more of claims 3 to 4, wherein said at least one control and command unit (5) is configured to send an alarm signal to a device locally or remotely, if said control and command unit (5) does not detect at least one detection signal stored beforehand or detects at least one detection signal not stored beforehand.

6. Machine according to one or more of the previous claims, wherein said at least one component (2) is movable along a predetermined path (P).

7. Machine according to one or more of the previous claims, wherein said at least one identification device (4) is placed facing said at least one component (2) and/or said path (P).

8. Machine according to one or more of the previous claims, wherein said identification means (3, 3') are applied in a removable way to each component (2) or are integrated into each component (2).

9. Machine according to one or more of the previous claims, wherein said at least one identification device (4) comprises at least one sensor selected from the group consisting of: inductive sensor, radio frequency sensor, optical sensor, mechanical sensor, thermal sensor, proximity sensor, movement sensor.

10. Machine according to one or more of the previous claims, wherein said identification means (3, 3') comprise at least one element selected from the group consisting of: barcode, passive electronic devices, alphanumeric sequences.

11. Method for producing and/or packaging products made from cellulose or absorbent products, said absorbent products preferably comprising nappies, adult diapers, sanitary pads, drawsheets and plasters, said method comprising the following steps:
- providing at least one operating component (2) of a machine (1) comprising identification means (3, 3') connected or joined to said at least one component (2):
- providing at least one identification device (4) configured to detect at least one or more of said identification means (3, 3') if needed arranged on more components (2).

12. Method according to the previous claim, wherein said identification means (3, 3') are univocal for each component (2) and configured in such a way that said identification device (4) generates a univocal detection signal for each component (2) adapted to recognize each component (2).

13. Method according to claim 11 or 12, wherein said method comprises a step of providing at least one control and command unit (5) configured to store beforehand each univocal detection signal for each component (2) and to associate each univocal detection signal, subsequently detected, with each component (2).

14. Method according to claim 13, wherein said at least one control and command unit (5) is configured to act on at least one component of said machine (1) for stopping or preventing its functioning, if said control and command unit (5) does not detect at least one detection signal stored beforehand or detects at least one detection signal not stored beforehand.

15. Method according to any one or more of claims 13 to 14, wherein said at least one control and command unit (5) is configured to send an alarm signal to a device locally or remotely, if said control and command unit (5) does not detect at least one detection signal stored beforehand or detects at least one detection signal not stored beforehand.
